(19) Europäisches Patentamt
European Patent Office
Office européen des brevets

(11) Veröffentlichungsnummer: **0 185 621 B1**

(12) **EUROPÄISCHE PATENTSCHRIFT**

(45) Veröffentlichungstag der Patentschrift: **16.10.91**

(51) Int. Cl.⁵: **C07D 213/64**, A01N 47/34, //C07D213/61

(21) Anmeldenummer: **85810601.6**

(22) Anmeldetag: **16.12.85**

Die Akte enthält technische Angaben, die nach dem Eingang der Anmeldung eingereicht wurden und die nicht in dieser Patentschrift enthalten sind.

(54) **Benzoylphenylharnstoffe.**

(30) Priorität: **20.12.84 CH 6054/84**

(43) Veröffentlichungstag der Anmeldung: **25.06.86 Patentblatt 86/26**

(45) Bekanntmachung des Hinweises auf die Patenterteilung: **16.10.91 Patentblatt 91/42**

(84) Benannte Vertragsstaaten: **BE CH DE FR GB IT LI NL**

(56) Entgegenhaltungen:
**EP-A- 0 077 759**
**EP-A- 0 078 234**
**EP-A- 0 101 662**
**EP-A- 0 138 772**
**EP-A- 0 177 455**
**DE-A- 3 241 138**
**DE-A- 3 337 828**

**TETRAHEDRON, Band 41, Nr. 19, 1985, Seiten 4057-4078, Pergamon Press Ltd, GB; P. MARTIN et al.: "Convenient approaches to heterocycles via copper-catalysed additions of organic polyhalides to activated olefins"**

(73) Patentinhaber: **CIBA-GEIGY AG**
**Klybeckstrasse 141**
**CH-4002 Basel(CH)**

(72) Erfinder: **Böger, Manfred Wilhelm**
**Glockstrasse 14**
**W-7858 Weil am Rhein 5(DE)**
Erfinder: **Drabek, Jozef, Dr.**
**Benkenstrasse 12**
**CH-4104 Oberwil(CH)**
Erfinder: **Steiner, Eginhard, Dr.**
**Obere Höfackerstrasse 3**
**CH-4414 Füllinsdorf(CH)**

**EP 0 185 621 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft neue substituierte N-Benzoyl-N'-(2-pyridyloxyphenyl)-harnstoffe, Verfahren und Zwischenprodukte zu ihrer Herstellung und ihre Verwendung in der Schädlingsbekämpfung.

Die erfindungsgemässen substituierten N-Benzoyl-N'-(2-pyridyloxyphenyl)-harnstoffe haben die Formel I

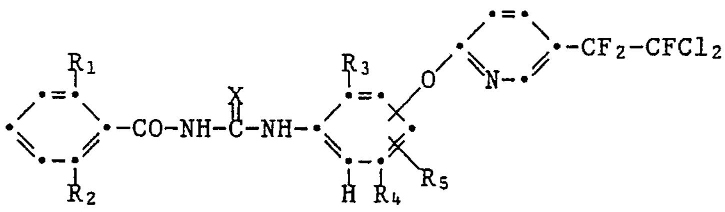

(I),

worin

$R_1$ Wasserstoff, Fluor oder Chlor;
$R_2$ Fluor, Chlor, Methyl, Methoxy oder Methylthio;
$R_3$, $R_4$ und $R_5$ unabhängig voneinander Wasserstoff, Chlor oder Methyl;
und
X Sauerstoff oder Schwefel
bedeuten.

Wegen ihrer Wirkung als Schädlingsbekämpfungsmittel bevorzugt sind solche Verbindungen der Formel I, die dadurch gekennzeichnet sind, dass X Sauerstoff bedeutet.

Hervorzuheben sind wirkungsmässig insbesondere diejenigen Verbindungen der Formel I, worin
$R_1$ Wasserstoff, Fluor oder Chlor;
$R_2$ Fluor oder Chlor;
$R_3$, $R_4$ und $R_5$ Wasserstoff; und
X Sauerstoff
bedeutet.

Die Verbindungen der Formel I können analog an sich bekannten Verfahren hergestellt werden (vgl. u.a. die deutschen Offenlegungsschriften Nr. 2.123.236, 2.601.780, und 3.240.975).

So kann man z.B. eine Verbindung der Formel I erhalten, durch Umsetzung

a) einer Verbindung der Formel II

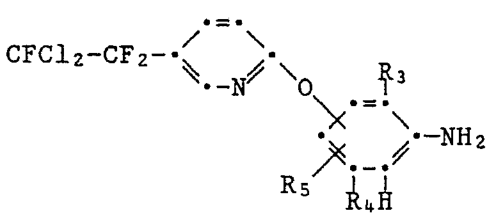

(II)

mit einer Verbindung der Formel III

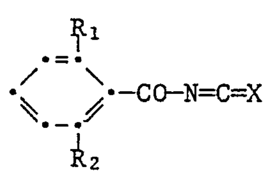

(III)

oder

b) einer Verbindung der Formel IV

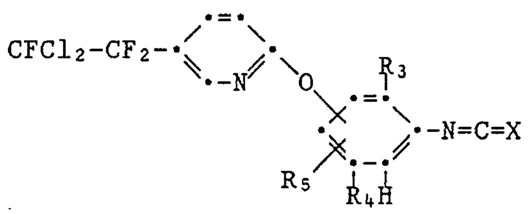

(IV)

gegebenenfalls in Gegenwart einer organischen oder anorganischen Base mit einer Verbindung der Formel V

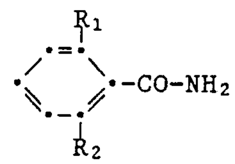

(V)

oder

c) einer Verbindung der Formel II mit einer Verbindung der Formel VI

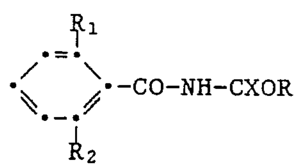

(VI).

In den obigen Formeln II bis VI haben die Reste $R_1$ bis $R_5$ und X die unter Formel I vorstehend angegebenen Bedeutungen; R bedeutet einen $C_1$-$C_8$-Alkylrest, der gegebenenfalls mit Halogen, vorzugsweise Chlor, substituiert ist.

Die erwähnten Verfahren a), b) und c) werden vorzugsweise unter normalem Druck und in Gegenwart eines organischen Lösungs- oder Verdünnungsmittels durchgeführt. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von -10 bis 200 °C, vorzugsweise zwischen 50 und 150 °C, gegebenenfalls in Gegenwart einer organischen Base, z.B. Triäthylamin, durchgeführt. Die Durchführung von Verfahren b) erfolgt bei einer Temperatur von 0 bis 150 °C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium. Für das Verfahren c), d.h. für die Umsetzung der Urethane der Formel VI mit einem Pyridyloxyanilin der Formel II, werden Temperaturen zwischen etwa 60 °C und dem Siedepunkt des jeweiligen Reaktionsgemisches bevorzugt, wobei als Lösungsmittel insbesondere aromatische Kohlenwasserstoffe, wie Toluol, Xylole, Chlorbenzol usw., verwendet werden.

Die Ausgangsstoffe der Formeln III, V und VI sind bekannt und können analog bekannten Verfahren hergestellt werden. Bei den Ausgangsstoffen der Formel II handelt es sich um neuartige Verbindungen, die ebenfalls einen Gegenstand der vorliegenden Erfindung bilden. Man kann diese Pyridyloxyaniline der Formel II herstellen durch Umsetzung entsprechender Aminophenole der Formel VII

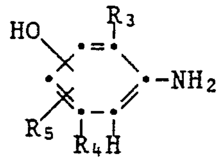

(VII)

mit 2-Halogen-5-halogenäthyl-pyridinen der Formel VIII

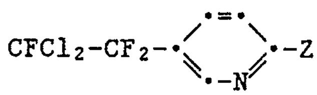

(VIII)

oder durch Umsetzung entsprechender p-Nitrophenole der Formel IX mit Pyridinen der Formel VIII und anschliessende Reduktion der Nitrogruppe in den entstehenden Pyridyloxynitrobenzolen der Formel X nach einem der üblichen Verfahren [vgl. z.B. Rec. 21, 271 (1902); J.Am. Soc. 68, 1604 (1946); J. Org. Chem. 11, 378 (1946); Rec. 79, 995 (1960)]:

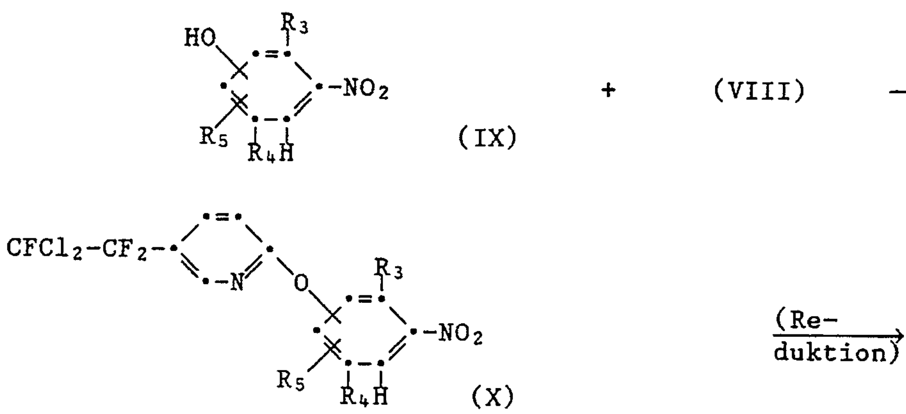


(IX) + (VIII) → (X) → (II)

In den obigen Formeln VII bis X haben $R_3$, $R_4$ und $R_5$ die vorstehend unter Formel I angegebenen Bedeutungen; Z bedeutet Fluor, Chlor oder Brom, vorzugsweise Chlor.

Zu Benzoylisocyanaten gemäss Formel III kann man unter anderem wie folgt gelangen (vgl. J. Agr. Food Chem. 21, 348 und 993; 1973):

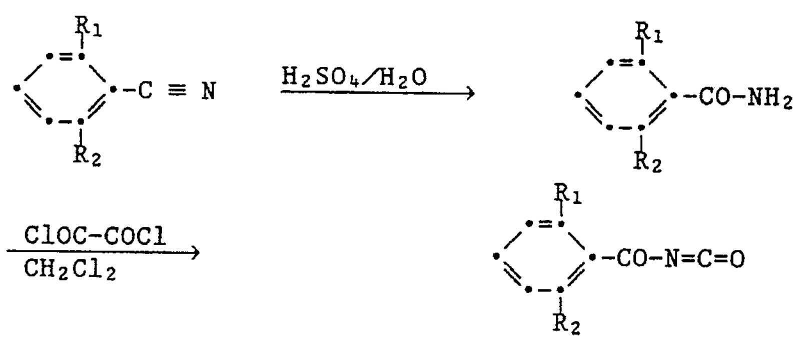


(III)

Die Benzoylisothiocyanate der Formel III können durch Umsetzung eines reaktionsfähigen Benzoylderivates, z.B. eines entsprechend substituierten Benzoylhalogenids, mit einem Isothiocyanat, z.B. KSCN, in allgemein üblicher Weise hergestellt werden.

Die Pyridyloxy-phenylisocyanate der Formel IV lassen sich z.B. durch Phosgenierung entsprechender Pyridyloxy-aniline der Formel II nach allgemein üblichen Verfahren hersstellen. Die weiterhin als Ausgangsstoffe zu verwendenden Benzamide der Formel V sind bekannt (vgl. z.B. Beilstein "Handbuch der organischen Chemie" Bd. 9, S. 336).

Urethane bzw. Thiourethane der Formel VI können in an sich bekannter Weise erhalten werden durch Umsetzung eines Benzoylisocyanats bzw. Benzoylisothiocyanats der Formel III mit einem entsprechenden Alkohol. Urethane der Formel VI sind ferner herstellbar durch Umsetzung eines Benzamides der Formel V in Anwesenheit einer basischen Verbindung mit einem entsprechenden Ester der Chlorameisensäure.

Die 2-Halogen-5-halogenäthyl-pyridine der Formel VIII sind ebenfalls neue Verbindungen und bilden einen Gegenstand der vorliegenden Erfindung. Die Herstellung der Pyridine der Formel VIII erfolgt hauptsächlich in an sich bekannter Weise (vgl. z.B. Europäische Patentanmeldung Nr. 0.078.234). Pyridine der Formel VIII können wie folgt hergestellt werden: Zunächst erfolgt Perchlorierung der Aethylgruppe in einer Verbindung der Formel XI

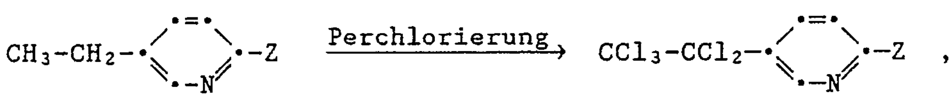


(XI) (XIa)

wobei Z Fluor, Chlor oder Brom bedeutet (vgl. C.A. 57:790, 1962; 69:86786r, 1968). Diese Perchlorierung kann in flüssiger Phase mit oder ohne Lösungsmittel in Gegenwart von Licht und/oder anderen Radikalbildnern, wie z.B. Azo-isobutyronitril oder Benzoylperoxid, bei Raumtemperatur aber auch bei erhöhten Temperaturen z.B. von 40 bis ca. 100 °C, durchgeführt werden. Die Menge des Radikalbildners kann von 0,001 Mol bis 1, Mol pro Mol zu chlorierende Substanz variieren. Die Reaktionstemperatur liegt im allgemeinen im Bereich zwischen Raumtemperatur und ca. 150 °C, vorzugsweise ist sie identisch mit dem Siedepunkt des verwendeten Lösungsmittels. Als Lösungsmittel werden solche bevorzugt die gegen Chlor inert sind. Die Ausgangsmaterialien, d.h. die 2-Halogen-5-äthylpyridine der Formel XI, können als freie Pyridine oder als deren Hydrochloride bzw. Hydrobromide eingesetzt werden. Der Verlauf der Chlorierungsreaktion kann mit Hilfe der Gaschromatographie oder der Dünnschichtchromatographie verfolgt werden. Die Aufarbeitung der Reaktionslösung erfolgt nach Austreiben gelösten Halogenwasserstoffs bzw. überschüssigen Chlors mittels eines Stickstoffstromes durch Eindampfen der Lösung. Das erhaltene Rohprodukt der Formel XIa kann durch Umkristallisation gereinigt oder für den erwünschten Austausch von Chlor gegen Fluor auch direkt weiterverarbeitet werden.

Dieser Austausch von Chlor gegen Fluor in der $-CCl_2-CCl_3$-Gruppe eines erhaltenen Pyridins der Formel XIa unter Bildung von entsprechenden Pyridinen der Formel VIII kann mit einem Fluorierungsmittel, wie z.B. KF, NaF, $CsF_2$, $SbF_3$, HF oder deren Gemischen, durchgeführt werden. Die Reaktion wird oft durch geringe Mengen von elementarem Chlor katalysiert und wird entweder in der Schmelze oder in einem Lösungsmittel bei Temperaturen von 50 ° bis 250 °C durchgeführt. Im Falle der Umsetzung mit HF wird die Reaktion vorteilhaft unter erhöhtem Druck in einem Autoklaven vollzogen. Als Lösungsmittel eignen sich polare aprotische Verbindungen, wie z.B. Dimethylformamid, Dimethylacetamid, Sulfolan, Dimethylsulfoxid oder Dimethyl- oder Diäthylsulfon. Die Fluorierungsmittel werden bevorzugt in wasserfreier, feinverteilter Form und im Ueberschuss zu den auszutauschenden Chloratomen eingesetzt; dieser Ueberschuss beträgt üblicherweise 10 bis 200 %. Im Falle der Verwendung eines Lösungsmittels ist es vorteilhaft, einen Phasentransferkatalysator, wie z.B. Tetraalkylammonium-chlorid oder -bromid, in Mengen von 0,1 - 10 % des Lösungsmittels dem Reaktionsgemisch zuzusetzen.

Aus der europäischen Patentanmeldung 0 077 759 und der deutschen Offenlegungsschrift 27 48 636 sind bereits unterschiedlich substituierte N-Benzoyl-N'-(2-pyrid-4-yloxyphenyl)-harnstoffe mit insektizider Wirkung bekannt. Aehnlich substituierte, pestizid wirksame N-Benzoyl-N'-(2-pyridyloxyphenyl)-thioharnstoffe werden in der DE-OS 3 337 828 beschrieben. Die erfindungsgemässen Verbindungen der Formel I unterscheiden sich strukturell demgegenüber im wesentlichen dadurch, dass sie eine in 3-Stellung unsubstituierte Pyridyloxygruppe aufweisen, die in 5-Stellung mit einem perhalogenierten Aethylrest substituiert ist. Ferner sind pestizid wirksame N-Benzoyl-N'-[4-{5'-(2,2-dichlor-1,1,2-trifluoräthyl)-pyridyl-2'-oxy}-3-carbalkoxy-phenyl]-harnstoffe aus der nach dem Anmeldetag des vorliegenden Patentes veröffentlichten europäischen Patentanmeldung 0.177.455 bekannt.

Ueberraschenderweise wurde gefunden, dass die vorliegenden Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Insekten und Vertretern der Ordnung Akarina.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, sowie von Vertretern der Ordnung Akarina der Familien: Ixodidae, Argasidae, Tetranychidae und Dermanyssidae.

Neben ihrer Wirkung gegenüber Fliegen, wie z.B. Musca domestica, und Mückenlarven können Verbindungen der Formel I auch zur Bekämpfung von pflanzenschädigenden Frassinsekten in Zier- und Nutzpflanzungen, insbesondere in Baumwollkulturen (z.B. Spodoptera littoralis und Heliothis virescens), sowie in Obst- und Gemüsekulturen (z.B. Laspeyresia pomonella, Leptinotarsa decemlineata und Epilachna varivestis) eingesetzt werden. Die Verbindungen der Formel I zeichnen sich durch eine ausgeprägte larvizide und ovo-larvizide Wirkung gegen Insekten, insbesondere Larven von fressenden Schadinsekten, aus. Werden Verbindungen der Formel I von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I können ferner zur Bekämpfung von Ektoparasiten, wie Lucilia sericata, an Haus- und Nutztieren eingesetzt werden, z.B. durch Tier-, Stall- und Weidebehandlung.

Die Verbindungen der Formel I eignen sich ferner zur Bekämpfung der folgenden Obst- und Gemüsekulturen befallenden Milbenspezies: Tetranychus urticae, Tetranychus cinnabarinus, Panonychus ulmi, Broybia rubrioculus, Panonychus citri, Eriophyes piri, Eriophyes ribis, Eriophyes vitis, Tarsonemus pallidus, Phyllocoptes vitis und Phyllocoptruta oleivora.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyltaurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung der Ausgangs- und Zwischenprodukte

a) Herstellung von 2-Chlor-5-pentachloräthyl-pyridin:

14,1 g 2-Chlor-5-äthylpyridin werden in 200 ml Tetrachlorkohlenstoff gelöst. Unter Kühlung werden bei Raumtemperatur 6 g HCl-Gas eingeleitet, wobei teilweise das Hydrochlorid der Pyridinverbindung ausfällt. Das Reaktionsgemisch wird auf 50° C erwärmt und unter Belichtung mittels einer Hg-Hochdrucklampe (125 Watt) mit $Cl_2$-Gas behandelt. Das Fortschreiten der Chlorierungsreaktion wird

dünnschichtchromatographisch (Kieselgel als Träger; Toluol als Laufmittel) verfolgt. Wenn die Reaktion beendet ist, wird die hellgelbe Lösung im Vakuum eingedampft. Man erhält als Rückstand die Titelverbindung der Formel

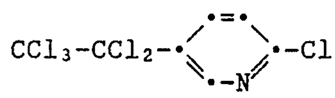

als gelbliches Produkt, das nach Umkristallisieren aus 90 %-igem Aethanol bei 92° C schmilzt.

b) Herstellung von 2-Chlor-5-(2,2-dichlor-1,1,2-trifluoräthyl)pyridin:

31,4 g 2-Chlor-5-pentachloräthyl-pyridin werden zusammen mit 90 g Antimontrifluorid und 4 g Antimonpentachlorid vermischt und auf 180° C erhitzt. Es entsteht eine dunkelbraune Schmelze, die während drei Stunden bei dieser Temperatur gehalten wird. Nach dem Abkühlen des Ansatzes auf 90° C fügt man dem Reaktionsgemisch 200 ml heisses Wasser zu und destilliert dann das Produkt mit Wasserdampf ab. Das wässrig-ölige Wasserdampf-Destillat wird mit Aether extrahiert, der Aetherextrakt mit $Na_2SO_4$ getrocknet und der Aether abdestilliert. Das zurückbleibende gelbliche Oel wird am Wasserstrahlvakuum rektifiziert. Das bei 94-97° C/15,5 mbar destillierende Produkt wird aufgefangen; es erstarrt beim Stehen zu weissen Kristallen. Die so erhaltene Titelverbindung der Formel

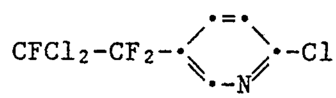

hat einen Schmelzpunkt von 38° C.

c) Herstellung von 2-Fluor-5-(2,2-dichlor-1,1,2-trifluoräthyl)pyridin:

Wird die Fluorierungsreaktion mit 2-Chlor-5-pentachloräthyl-pyridin analog der unter b) angegebenen Arbeitsweise während vier Stunden bei 200° C durchgeführt und der Ansatz anschliessend wie vorstehend unter b) angegeben aufgearbeitet, so erhält man die Titelverbindung der Formel

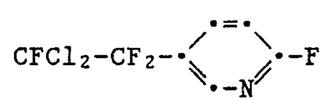

mit einem Siedepunkt von 70-71° C/14 mbar.

d) Herstellung von 4-[5-(2',2'-Dichlor-1',1',2'-trifluoräthyl)-2-pyridyloxy]-anilin:

Es werden 2,8 g 4-Aminophenol und 6,0 g Kaliumcarbonat in 30 ml Dimethylsulfoxid bei Raumtemperatur während 1 Stunde gerührt. Anschliessend wird der Ansatz auf 50° C erwärmt und eine Lösung von 6,15 g des gemäss b) erhaltenen 2-Chlor-5-(2,2-dichlor-1,1,2-trifluoräthyl)-pyridins in 10 ml Dimethylsulfoxid zugetropft. Das Reaktionsgemisch wird danach während 4 Stunden bei 95° C gerührt. Das Dimethylsulfoxid wird dann durch Eindampfen am Hochvakuum entfernt, der gebildete Rückstand in Dichlormethan und Wasser aufgenommen, die abgetrennte organische Phase getrocknet und stark eingeengt. Nach Filtration über Kieselgel, Eindampfen der Lösung und Zugabe von etwas Hexan kristallisiert die Titelverbindung der Formel

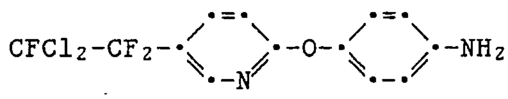

als helles Pulver mit einem Schmelzpunkt von 75-77 °C aus.

Analog den vorstehenden Arbeitsweisen sind auch folgende Verbindungen der Formel II herstellbar:

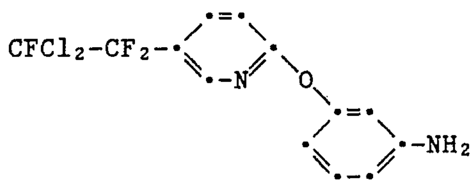

Smp. 57-9°C

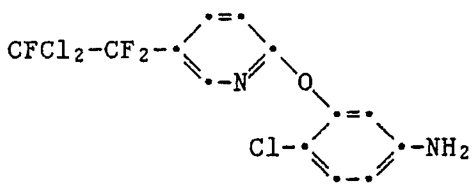

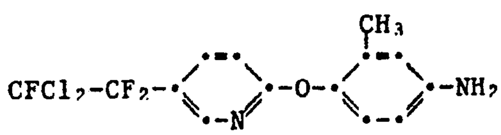

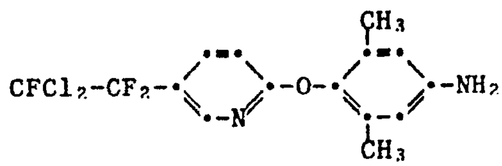

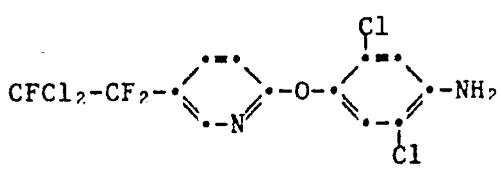

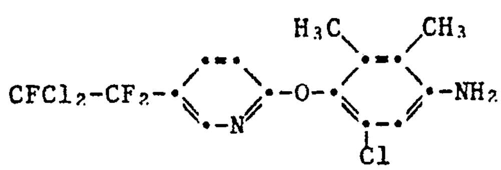

Beispiel 2: Herstellung von N-4-[5-(2',2'-Dichlor-1',1',2'-trifluoräthyl)-2-pyridyloxy]-phenyl-N'-2,6-difluorbenzoylharnstoff:

Es werden 2,25 g 4-[5-(2',2'-Dichlor-1',1',2'-trifluoräthyl)-2-pyridoxy]-anilin in 20 ml absolutem Toluol gelöst und unter Ausschluss von Feuchtigkeit langsam mit einer Lösung von 1,22 g 2,6-Difluor-benzoylisocyanat in 10 ml Toluol versetzt. Man lässt die exotherme Reaktion abklingen und rührt das Reaktionsgemisch während 5 Stunden bei Raumtemperatur nach. Der ausgefallene kristalline Niederschlag wird abgesaugt und in Methanol unter Zugabe von wenig Aceton umkristallisiert. Man erhält so die Titelverbindung der Formel

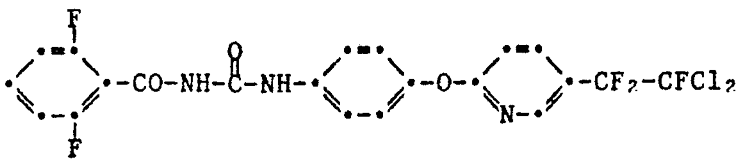

als hellbeiges kristallines Produkt vom Schmelzpunkt 207-208 °C

(Verbindung Nr. 1)

Entsprechend den vorstehend beschriebenen Arbeitsweisen werden die folgenden Verbindungen der Formel I hergestellt:

| Verbindung Nr. | | Smp. [°C] |
|---|---|---|
| 2 | 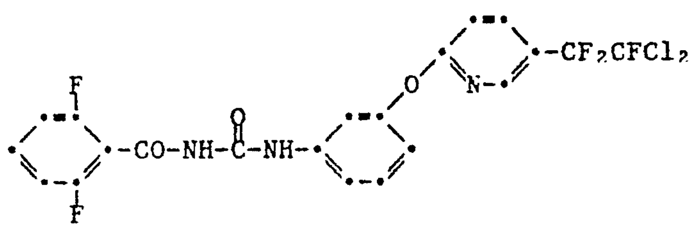 | 176-178 |
| 3 | 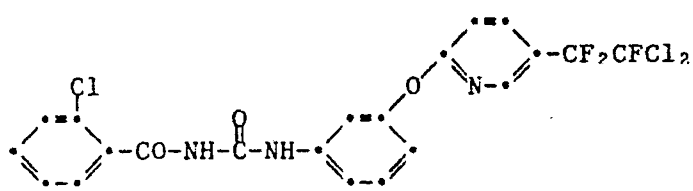 | 206-208 |
| 4 | 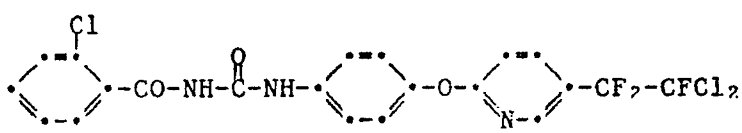 | 175-177 |
| 5 | 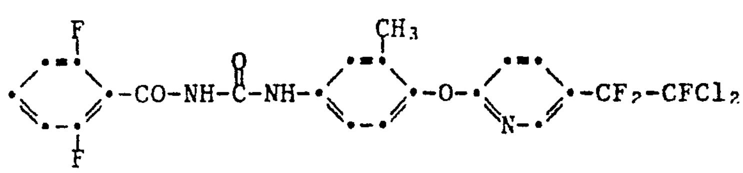 | 198-200 |

Analog den vorstehend aufgeführten Arbeitsweisen sind auch die folgenden Verbindungen der Formel I herstellbar:

Verbindung
Nr.

6 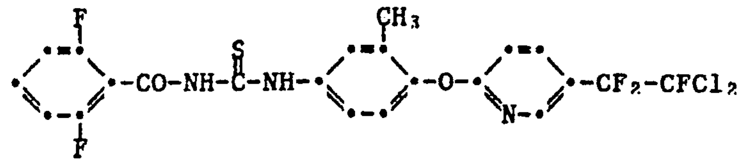

7 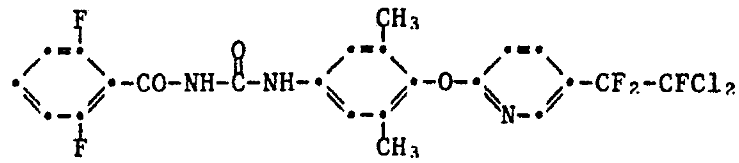

8 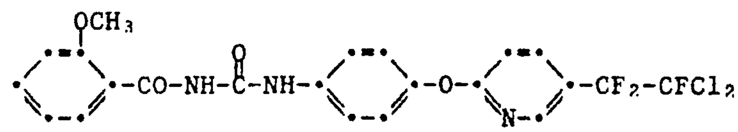

9 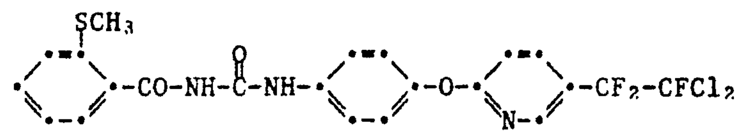

10 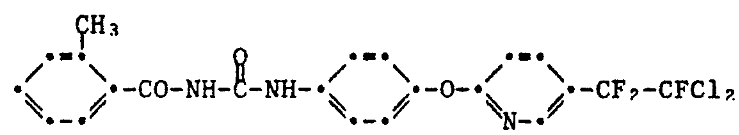

11 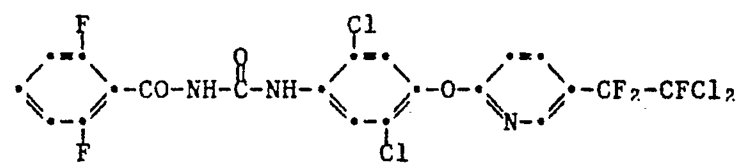

12 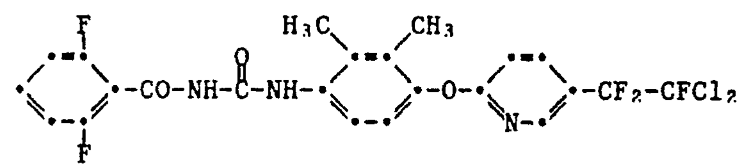

13 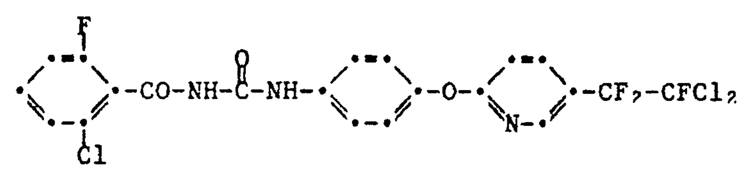

14

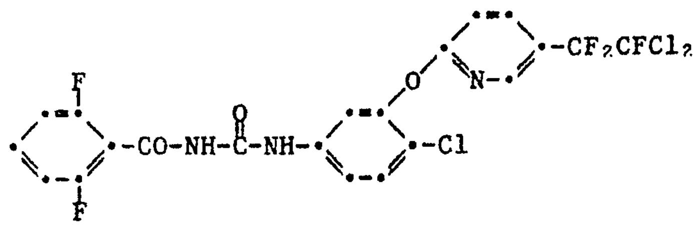

Beispiel 3:

Formulierungen für Wirkstoffe der Formel I gemäss Beispiel 2 resp. Kombinationen dieser Wirkstoffe mit andern Insektiziden oder Akariziden (% = Gewichtsprozent)

| 3.1 Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 3.2 Emulsions-Konzentrat | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3.3 Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff oder die Wirkstoffkombination mit dem Träger vermischt und auf einer geeigneten Mühle vermahlen wird.

| 3.4 Extruder-Granulat | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

| 3.5 Umhüllungs-Granulat | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

| 3.6 Suspensions-Konzentrat | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 4: Wirkung gegen Musca domestica:

Je 50 g frisch zubereitetes Nährsubstrat für Maden wird in Becher eingewogen. Von einer 1 Gew.-%igen acetonischen Lösung des betref-fenden Wirkstoffes wird eine bestimmte Menge auf das in den Bechern befindliche Nährsubstrat pipettiert, so dass sich eine Wirkstoffkonzentration von 800 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert. Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 2 zeigen gute Wirkung im obigen Test.

Beispiel 5: Wirkung gegen Lucilia sericata:

Zu 9 ml eines Zuchtmediums wird bei 50 °C 1 ml einer 0,5 Gew.-% Aktivsubstanz enthaltenden wässrigen Zubereitung hinzugefügt. Nun werden ca. 30 frisch geschlüpfte Lucilia sericata-Larven zum Zuchtmedium gegeben, und nach 48 und 96 Stunden wird die insektizide Wirkung durch Ermittlung der Abtötungsrate festgestellt.

Verbindungen der Formel I gemäss Beispiel 2 zeigen in diesem Test gute Wirkung gegen Lucilia sericata.

Beispiel 6: Wirkung gegen Aëdes aegypti:

Auf die Oberfläche von 150 ml Wasser, das sich in einem Behälter befindet, wird so viel einer 0,1 Gew.-%igen acetonischen Lösung des Wirkstoffes pipettiert, dass eine Konzentration von 800 ppm erhalten wird. Nach Verdunsten des Acetons wird der Behälter mit 30 bis 40 2-tägigen Aëdes-Larven beschickt. Nach 1, 2 und 5 Tagen wird die Mortalität geprüft.

Verbindungen der Formel I gemäss Beispiel 2 zeigen in diesem Test gute Wirkung gegen Aëdes aegypti.

Beispiel 7: Insektizide Frassgift-Wirkung:

Ca. 25 cm hohe eingetopfte Baumwollpflanzen werden mit wässrigen Wirkstoffemulsionen besprüht, die den Wirkstoff jeweils in ansteigenden Konzentrationen von 3 ppm bis zu 100 ppm enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24 °C und 60 % relativer Luftfeuchtigkeit durchgeführt. Nach 120 Stunden wird die %-Mortalität der Test-Insekten bestimmt.

Im obigen Test zeigen erfindungsgemässe Verbindungen 80-100%ige Wirksamkeit (Mortalität) bei folgenden Konzentrationen:

| Verbindung Nr. | Wirkstoffkonzentration [ppm] | |
|---|---|---|
| | Spodoptera | Heliothis |
| 2 | 3 ppm | 3 ppm |
| 3 | 50 ppm | 50 ppm |
| 4 | 12,5 ppm | 100 ppm |
| 5 | 50 ppm | - |

Beispiel 8: Wirkung gegen Epilachna varivestis:

Etwa 15-20 cm hohe Phaseolus vulgaris-Pflanzen (Buschbohnen) werden mit wässrigen, den zu prüfenden Wirkstoff in einer Konzentration von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Ueber die infestierten Pflanzen wird ein Plastikzylinder gestülpt, der mit einem Kupfer-Gazedeckel abgedeckt ist. Der Versuch wird bei 28° C und 60 % relativer Luftfeuchtigkeit durchgeführt.

Nach 2 und 3 Tagen wird die % Mortalität bestimmt. Zur Auswertung hinsichtlich allfälligem Frass-Schaden (Antifeeding-Effekt), Entwicklungs- und Häutungsstörungen werden die Versuchstiere während weiterer 3 Tage beobachtet.

Verbindungen der Formel I gemäss Beispiel 2 zeigen gute Wirkung im obigen Test.

Beispiel 9: Ovizide Wirkung auf Heliothis virescens:

Entsprechende Mengenanteile einer benetzbaren pulverförmigen Formulierung, enthaltend 25 Gew.% des zu prüfenden Wirkstoffes, werden mit jeweils soviel Wasser vermischt, dass sich wässrige Emulsionen mit einer Wirkstoffkonzentration von 800 ppm ergeben.

In diese wirkstoffhaltigen Emulsionen werden eintägige Eigelege von Heliothis auf Cellophan während drei Minuten eingetaucht und dann auf Rundfilter abgenutscht. Die so behandelten Gelege werden in Petrischalen ausgelegt und in der Dunkelheit aufbewahrt. Nach 6 bis 8 Tagen wird die Schlupfrate im Vergleich Zu unbehandelten Kontrollen festgestellt.

Verbindungen der Formel I gemäss Beispiel 2 zeigen gute Wirkung in obigem Test.

Beispiel 10: Wirkung auf Laspeyresia pomonella (Eier):

Abgelegte Eier von Laspeyresia pomonella, die nicht älter als 24 Stunden sind, werden auf Filterpapier für 1 Minute in eine acetonisch-wässrige Lösung, enthaltend 800 ppm des zu prüfenden Wirkstoffes, eingetaucht. Nach dem Antrocknen der Lösung werden die Eier in Petrischalen ausgelegt und bei einer Temperatur von 28° C belassen. Nach 6 Tagen wird der prozentuale Schlupf aus den behandelten Eiern bewertet und die %-Mortalität bestimmt.

Verbindungen der Formel I gemäss Beispiel 2 zeigen gute Wirkung in obigem Test.

Beispiel 11: Reproduktions-Beeinflussung von Anthonomus grandis:

Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 400 ppm des zu prüfenden Wirkstoffes, eingetaucht. Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraums von etwa 4 Wochen überprüft. Die Bonitierung erfolgt anhand der Verminderung der Anzahl abgelegter Eier und der daraus geschlüpften Larven im Vergleich zu unbehandelten Kontrollen.

Die Verbindung Nr. 1 gemäss Beispiel 2 zeigt eine 80-100%-ige reproduktionsreduzierende Wirkung im obigen Test.

Beispiel 12: Wirkung gegen Anthonomus grandis (Adulte)

Zwei eingetopfte Baumwollpflanzen im 6-Blattstadium werden jeweils mit einer wässrigen benetzungsfähigen Emulsions-Zubereitung enthaltend 400 ppm des zu prüfenden Wirkstoffes besprüht. Nach dem Antrocknen des Spritzbelages (nach etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die so behandelten Pflanzen werden bei 25° C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen der Formel I gemäss Beispiel 2 zeigen gute Wirkung in obigem Test.

## Patentansprüche

1. Verbindung der Formel I,

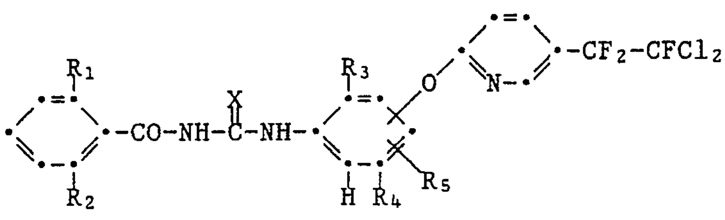

(I),

worin
$R_1$ Wasserstoff, Fluor oder Chlor;
$R_2$ Fluor, Chlor, Methyl, Methoxy oder Methylthio;
$R_3$, $R_4$ und $R_5$ unabhängig voneinander Wasserstoff, Chlor oder Methyl; und
X Sauerstoff oder Schwefel
bedeuten.

2. Verbindung der Formel I gemäss Anspruch 1 oder 2, dadurch gekennzeichnet, dass X Sauerstoff bedeutet.

3. Verbindung der Formel I gemäss Anspruch 1, dadurch gekennzeichnet, dass
$R_1$ Wasserstoff, Fluor oder Chlor;
$R_2$ Fluor oder Chlor;
$R_3$, $R_4$ und $R_5$ Wasserstoff; und
X Sauerstoff
bedeuten.

4. Verbindung gemäss Anspruch 3 der Formel

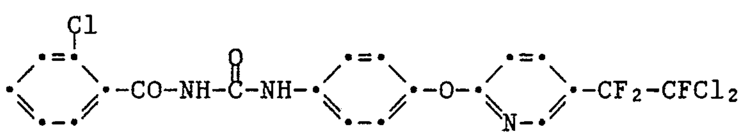

5. Verbindung gemäss Anspruch 3 der Formel

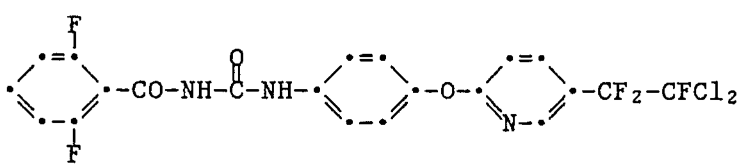

6. Verbindung gemäss Anspruch 3 der Formel

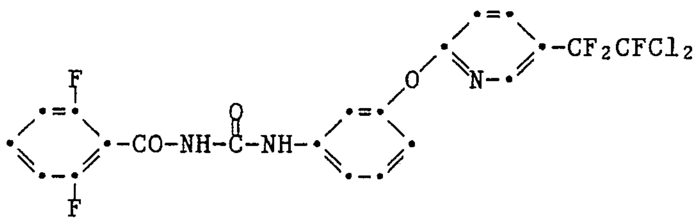

**7.** Verbindung gemäss Anspruch 3 der Formel

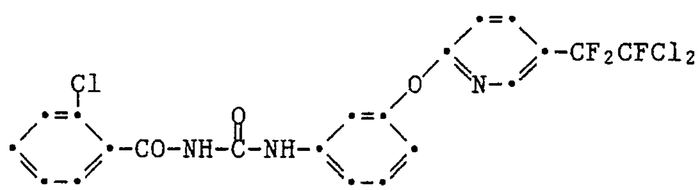

**8.** Verfahren zur Herstellung einer Verbindung gemäss einem der Ansprüche 1 bis 7, dadurch gekennzeichnet, dass man

eine Verbindung der Formel II

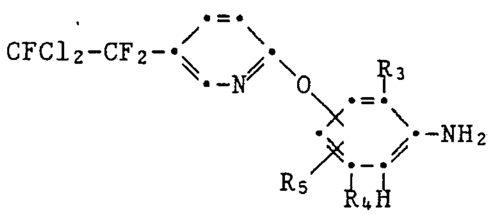

(II)

mit einer Verbindung der Formel III

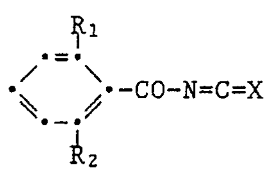

(III)

oder

b) eine Verbindung der Formel IV

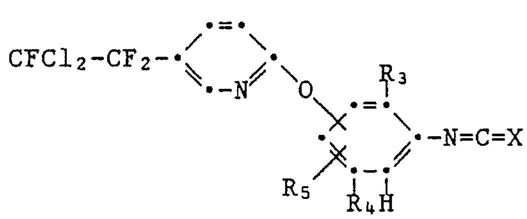

(IV)

mit einer Verbindung der Formel V

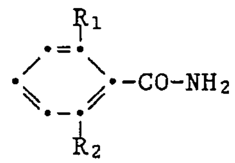

(V)

oder

c) eine Verbindung der Formel II mit einer Verbindung der Formel VI

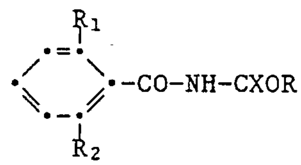

(VI)

umsetzt, wobei in den Formeln II bis VI die Reste $R_1$ bis $R_5$ sowie X die in den Ansprüchen 1 bis 3 angegebenen Bedeutungen haben und R für einen $C_1$-$C_8$-Alkylrest, der gegebenenfalls mit Halogen, vorzugsweise Chlor, substituiert ist, steht.

**9.** Verbindung der Formel II

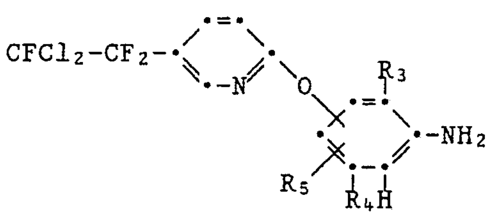

(II),

worin

$R_3$, $R_4$ und $R_5$ unabhängig voneinander Wasserstoff, Chlor oder Methyl bedeuten.

**10.** Verfahren zur Herstellung einer Verbindung der Formel II gemäss Anspruch 9, dadurch gekennzeichnet, dass man eine Verbindung der Formel VII

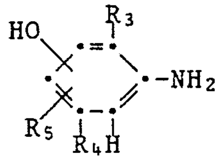

(VII)

mit einer Verbindung der Formel VIII

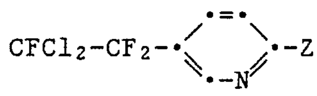

(VIII)

umsetzt, wobei $R_3$, $R_4$ und $R_5$ die in Anspruch 9 angegebenen Bedeutungen haben und Z für Fluor, Chlor oder Brom steht.

**11.** Verbindung der Formel VIII

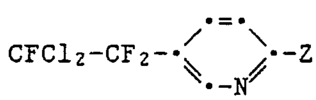

(VIII),

worin
Z Fluor, Chlor oder Brom
bedeutet.

**12.** Verbindung der Formel VIII gemäss Anspruch 11, dadurch gekennzeichnet, dass
Z Chlor
bedeutet.

**13.** Verfahren zur Herstellung einer Verbindung der Formel VIII gemäss einem der Ansprüche 11 oder 12, dadurch gekennzeichnet, dass man eine Verbindung der Formel (XI)

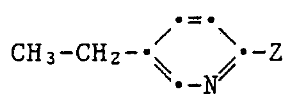

(XI),

chloriert unter Bildung einer Verbindung der Formel XIa

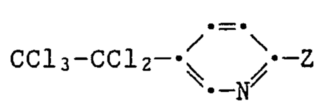

(XIa),

wobei Z Fluor, Chlor oder Brom bedeutet, und dass man in der erhaltenen Verbindung der Formel XIa mit einem Fluorierungsmittel Chloratome durch Fluoratome austauscht.

**14.** Verfahren gemäss Anspruch 13 zur Herstellung einer Verbindung der Formel VIII, dadurch gekennzeichnet, dass man als Fluorierungsmittel KF, NaF, $CsF_2$, $SbF_3$, HF oder Gemische dieser Verbindungen verwendet.

**15.** Verfahren gemäss Anspruch 13, dadurch gekennzeichent, dass man die Umsetzung in Gegenwart eines Lösungsmittels und eines Phasentransferkatalysators durchführt.

**16.** Schädlingsbekämpfungsmittel, welches als aktive Komponente eine Verbindung gemäss einem der Ansprüche 1 bis 7 zusammen mit geeigneten Trägern und/oder anderen Zuschlagstoffen enthält.

**17.** Verwendung einer Verbindung gemäss einem der Ansprüche 1 bis 7 zur Bekämpfung von Insekten und Vertretern der Ordnung Akarina.

**18.** Verwendung gemäss Anspruch 17 zur Bekämpfung larvaler Stadien pflanzenschädigender Insekten.

**19.** Verfahren zur Bekämpfung von Insekten und Vertretern der Ordnung Akarina, dadurch gekennzeichnet, dass man die Schädlinge bzw. deren verschiedene Entwicklungsstadien oder deren Aufenthaltsort mit einer pestizid wirksamen Menge einer Verbindung der Formel I gemäss einem der Ansprüche 1 bis 7 oder mit einem Mittel enthaltend neben Zusatz- und Trägerstoffen eine pestizid wirksame Menge dieser Verbindung, in Kontakt bringt oder behandelt.

## Claims

**1.** A compound of formula I

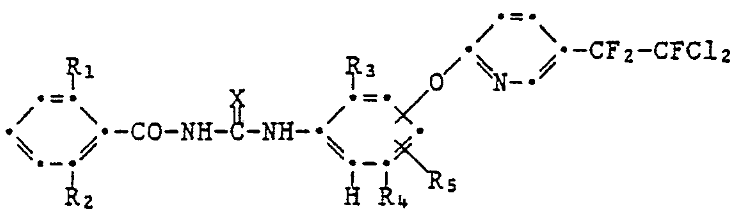

(I)

in which
$R_1$ is hydrogen, fluorine or chlorine; $R_2$ is fluorine, chlorine, methyl, methoxy or methylthio; $R_3$, $R_4$ and $R_5$ are each independently hydrogen, chlorine or methyl; and X is oxygen or sulphur.

**2.** A compound of formula I according to either of claims 1 and 2, wherein X is oxygen.

**3.** A compound of formula I according to claim 1, wherein $R_1$ is hydrogen, fluorine or chlorine; $R_2$ is fluorine or chlorine; $R_3$, $R_4$ and $R_5$ are hydrogen; and X is oxygen.

**4.** A compound according to claim 3 of the formula

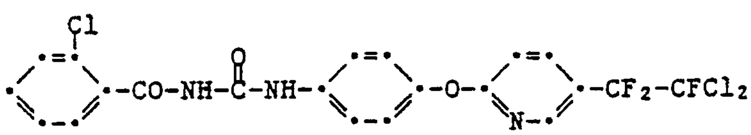

**5.** A compound according to claim 3 of the formula

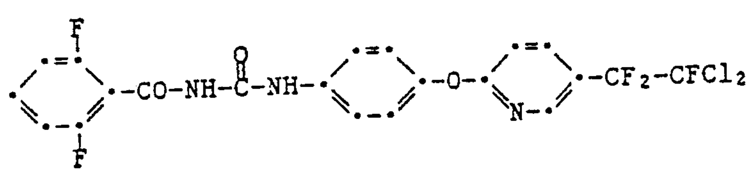

**6.** A compound according to claim 3 of the formula

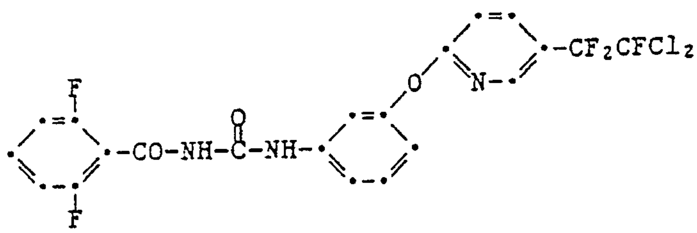

7. A compound according to claim 3 of the formula

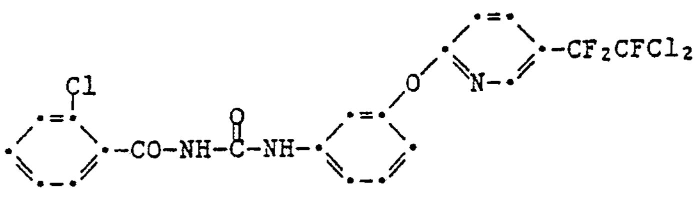

8. A process for the preparation of a compound according to any one of claims 1 to 7, which comprises
a) reacting a compound of formula II

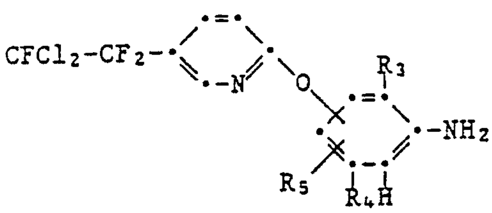

(II)

with a compound of formula III

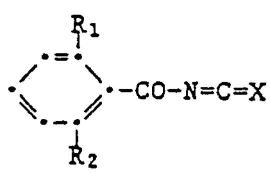

(III)

or
b) reacting a compound of formula IV

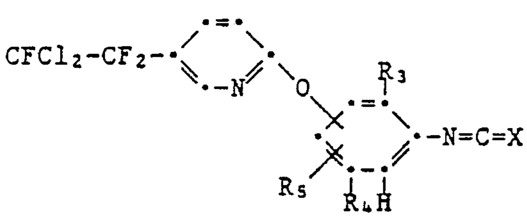

(IV)

with a compound of formula V

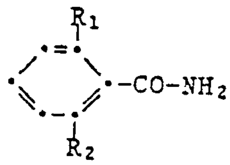

(V)

or

c) reacting a compound of formula II with a compound of formula VI

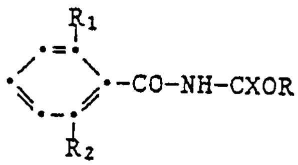

(VI)

where, in the formulae II to VI the radicals $R_1$ to $R_5$ and X are as defined in any one of claims 1 to 3 and R is a $C_1$-$C_8$alkyl radical which is unsubstituted or substituted by halogen, preferably chlorine.

**9.** A compound of formula II

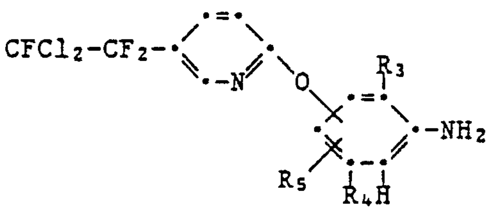

(II)

in which $R_3$, $R_4$ and $R_5$ are each independently hydrogen, chlorine or methyl.

**10.** A process for the preparation of a compound of formula II according to claim 9, which comprises reacting a compound of formula VII

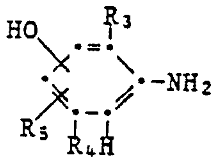

(VII)

with a compound of formula VIII

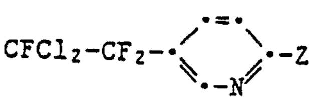

(VIII)

where $R_3$, $R_4$ and $R_5$ are as defined in claim 9 and Z is fluorine, chlorine or bromine.

**11.** A compound of formula VIII

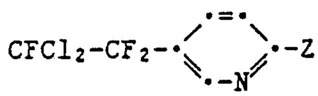

(VIII)

in which Z is fluorine, chlorine or bromine.

**12.** A compound of formula VIII according to claim 11, wherein Z is chlorine.

**13.** A process for the preparation of a compound of formula VIII according to either of claims 11 and 12, which comprises chlorinating a compound of formula XI

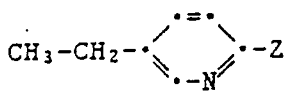

(XI)

to form a compound of formula XIa

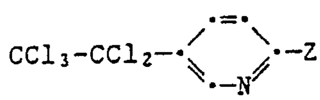

(XIa)

where Z is fluorine, chlorine or bromine and replacing chlorine atoms in the resultant compound of formula XIa by fluorine atoms using a fluorinating agent.

**14.** A process according to claim 13 for the preparation of a compound of formula VIII, which comprises the use of KF, NaF, $CsF_2$, $SbF_3$, HF or of a mixture of these compounds as fluorinating agent.

**15.** A process according to claim 13, which comprises carrying out the reaction in the presence of a solvent and of a phase transfer catalyst.

**16.** A pesticide which contains as active component a compound according to any one of claims 1 to 7, together with suitable carriers and/or other adjuvants.

**17.** Use of a compound according to any one of claims 1 to 7 for controlling insects and representatives of the order Acarina.

**18.** Use according to claim 17 for controlling larval stages of plant-destructive insects.

**19.** A method of controlling insects and representatives of the order Acarina, which comprises contacting or treating the pests, their various development stages or their locus with a pesticidally effective amount of a compound of formula I according to any one of claims 1 to 7, or with a composition which contains a pesticidally effective amount of this compound together with adjuvants and carriers.

**Revendications**

**1.** Composé de formule I

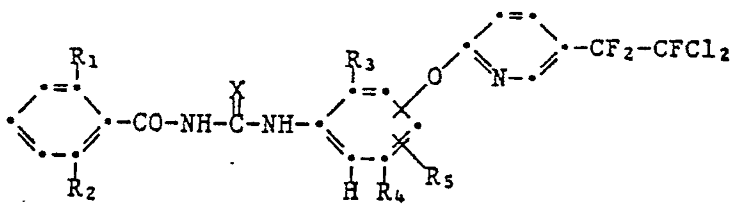 (I),

où
$R_1$ représente l'hydrogène, le fluor ou le chlore ;
$R_2$ représente le fluor, le chlore, le méthyle, le méthoxy ou le méthylthio ;
$R_3$, $R_4$ et $5_5$ représentent indépendamment les uns des autres, le chlore ou le méthyle ; et
X représente l'oxygène ou le soufre.

**2.** Composé de formule I selon la revendication 1, caractérisé en ce que X représente l'oxygène.

**3.** Composé de formule I selon la revendication 1, caractérisé en ce que $R_1$ représente l'hydrogène, le fluor ou le chlore ; $R_2$ le fluor ou le chlore ; $R_3$, $R_4$ et $R_5$ l'hydrogène ; et X l'oxygène.

**4.** Composé selon la revendication 3 de formule

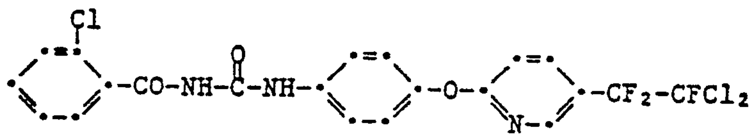

**5.** Composé selon la revendication 3 de formule

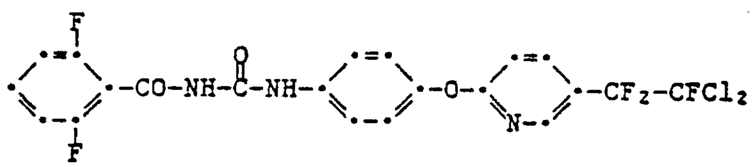

**6.** Composé selon la revendication 3 de formule

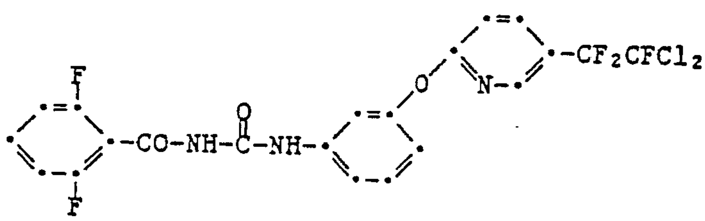

**7.** Composé selon la revendication 3 de formule

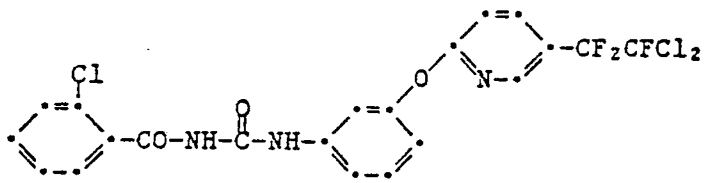

8. Procédé pour la préparation d'un composé selon l'une des revendications 1 à 7, caractérisé en ce que l'on fait réagir

a) un composé de formule II

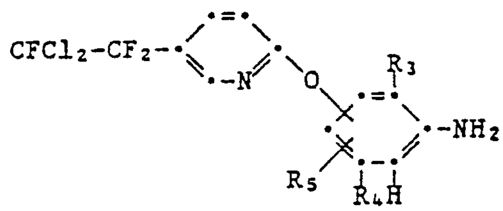

(II)

avec un composé de formule III

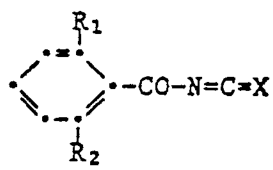

(III)

ou

b) un composé de formule IV

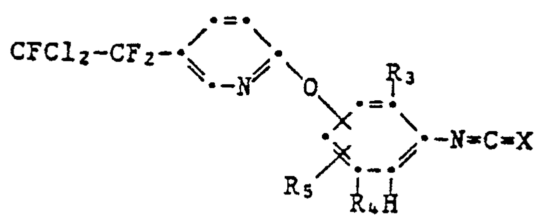

(IV)

avec un composé de formule V ou

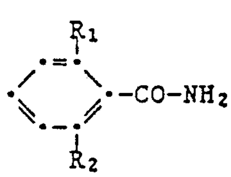

(V)

c) un composé de formule II avec un composé de formule VI

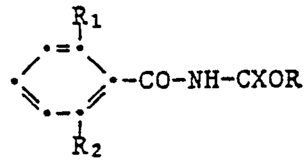

(VI)

les restes $R_1$ à $R_5$ ainsi que X ayant dans les formules II à VI les significations données dans les revendications 1 à 3 et R représentant un reste alkyle en $C_1$-$C_8$, éventuellement substitué par un halogène, de préférence le chlore.

**9.** Composé de formule II

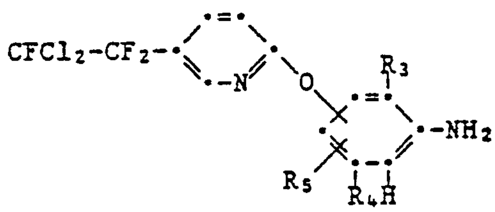

(II),

où
$R_3$, $R_4$ et $R_5$ représentent indépendamment les uns des autres l'hydrogène, le chlore ou le méthyle.

**10.** Procédé pour la préparation d'un composé de formule II selon la revendication 9, caractérisé en ce que l'on fait réagir un composé de formule VII

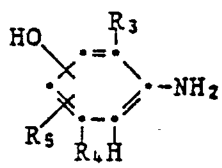

(VII)

avec un composé de formule VIII

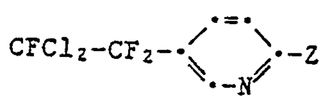

(VIII)

$R_3$, $R_4$ et $R_5$ ayant les significations données dans la revendication 9 et Z représentant le fluor, le chlore ou le brome.

**11.** Composé de formule VIII

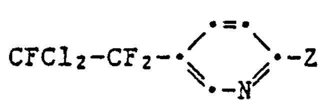

(VIII),

où

Z représente le fluor, le chlore ou le brome.

**12.** Composé de formule VIII selon la revendication 11, caractérisé en ce que Z représente le chlore.

**13.** Procédé pour la préparation d'un composé de formule VIII selon la revendication 11 ou 12, caractérisé en ce que l'on chlore un composé de formule (XI)

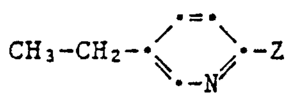

(XI),

un composé de formule XIa se formant

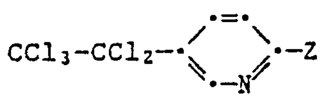

(XIa),

Z représentant le fluor, le chlore ou le brome et en ce que l'on échange dans le composé de formule XIa obtenu les atomes de chlore contre des atomes de fluor par un agent de fluoration.

**14.** Procédé selon la revendication 13 pour la préparation d'un composé de formule VIII, caractérisé en ce que l'on utilise comme agent de fluoration KF, NaF, $CsF_2$, $SbF_3$, HF ou des mélanges de ces composés.

**15.** Procédé selon la revendication 13, caractérisé en ce que l'on effectue la réaction en présence d'un solvant et d'un catalyseur de transfert de phases.

**16.** Agent de lutte contre les parasites contenant comme composant actif un composé selon l'une des revendications 1 à 7, associé à des supports appropriés et/ou à d'autres additifs.

**17.** Utilisation d'un composé selon l'une des revendications 1 à 7 pour la lutte contre les insectes et les représentants de l'ordre des acariens.

**18.** Utilisation selon la revendication 17 pour la lutte contre les stades larvaires des insectes nuisibles aux plantes.

**19.** Procédé pour lutter contre les insectes et représentants de l'ordre des acariens, caractérisé en ce que l'on met en contact ou en ce que l'on traite les parasites ou leurs différents stades de développement ou les endroits où ils séjournent avec une quantité efficace comme pesticide d'un composé de formule I selon l'une des revendications 1 à 7 ou avec un produit contenant à côté des additifs et supports une quantité efficace comme pesticide de ce composé.